# EUROPEAN PATENT SPECIFICATION

(11) **EP 3 061 432 B1**
(45) Date of publication and mention of the grant of the patent: **28.03.2018**
(21) Application number: 16164979.3
(22) Date of filing: 12.04.2016
(51) Int. Cl.: A61F 13/49, A61F 13/494, A61F 13/56, A61F 13/15

(54) **METHOD FOR PRODUCING ABSORBENT ARTICLE**
VERFAHREN ZUR HERSTELLUNG EINES SAUGFÄHIGEN ARTIKELS
PROCÉDÉ DE PRODUCTION D'ARTICLES ABSORBANTS

(30) Priority: 08.04.2016 JP 2016078479
(43) Date of publication of application: 31.08.2016
(73) Proprietor: Unicharm Corporation, Ehime 799-0111 (JP)
(72) Inventor: Morizane, Naoto, Kanonji-shi, Kagawa 769-1602 (JP); Seto, Hidenao, Kanonji-shi, Kagawa 769-1602 (JP)
(74) Representative: Dolleymores

(56) References cited:
- EP-A1- 2 474 494
- WO-A1-2005/110321
- US-A1- 2015 032 075

## Description

### Technical Field

The present invention relates to method for producing an absorbent article.

### Background Art

Absorbent articles such as disposable diapers are known. When such an absorbent article is produced, a folding step is carried out in which the absorbent article is folded for packaging of the absorbent article. In the folding step, a semi-processed continuous body, that is composed of semi-processed products for the absorbent article connected in the machine direction, is transported in the machine direction while both ends of the semi-processed product in the widthwise direction are folded from the outside to the inside. That is, side section regions on both outer sides of the center region in the widthwise direction of the semi-processed product are folded so as to overlap in the center region.

The folding method includes, for example, a method by a folding apparatus disclosed in JP2011-50478. The folding apparatus is a folding apparatus that folds a web that is being transported in a machine direction along a flow direction in a production process for the absorbent article. The folding apparatus comprises a rotor that presses the web in the direction crossing a surface of the web while feeding the web in the machine direction, and a guide section that guides side section regions of the web along the machine direction so as to overlay them over an inner side region of the web. Another folding method is disclosed in WO2010/092935, for example, as a method for producing a disposable wearable article. This method for producing a disposable wearable article comprises a first folding step in which a waist section is folded so that the non-skin side of the waist section overlaps with the non-skin side of side flaps during the transport step, and a second folding step in which the side flaps are folded in such a manner that skin sides of the side flaps overlap with skin sides of a back section or a front section after the first folding step during the transport step.

Further prior art arrangements are known from US2015/032075 and EP2474494.

### SUMMARY OF INVENTION

### Technical Problem

In some absorbent articles, a pair of protrusion sections (for example, back side flaps) are disposed protruding toward the outer side in the widthwise direction at both ends in the widthwise direction of the absorbent article. For production of such an absorbent article, in the above-mentioned folding step, there is supplied a semi-processed continuous body having a pair of protrusion sections that are not continuous in the machine direction, i.e., discontinuous, at each outer edge of a pair of side section regions connected in the machine direction.

When such a semi-processed continuous body is folded using the folding apparatus of Patent Literature 1, the side section regions that are continuous in the machine direction are easily folded so as to overlap the center region, but each of the protrusion sections that are not connected in the machine direction, i.e., that are discontinuous, is difficult to fold in a manner overlapping the center region. This is because the folding apparatus of Patent Literature 1 is designed for a product in which the side section regions to be folded are continuous in the lengthwise direction, and not for a structure having discontinuous portions (protrusion sections) in the side section regions. For example, focusing on specific sections in the side section regions that are continuous in the machine direction, other sections that are adjacent to those specific sections in the machine direction and are folded first facilitate the folding operation of those specific sections, so that the specific sections are easily folded in a manner overlapping the center region. However, since the adjacent protrusion sections in the machine direction are disposed in a mutually separated manner, that is, since the protrusion sections are disposed in a discontinuous manner, the leading protrusion sections do not facilitate the folding operation for the trailing protrusion sections. Consequently, when the side section regions are folded toward the center region so as to overlap the center region, the protrusion sections may or may not be able to follow the operation, and the manner of folding can potentially be unstable. In other words, with the folding apparatus described in Patent Literature 1, when the side section regions are folded, it can potentially be difficult to stably fold the protrusion sections. When this occurs, and the folded portions are opened during use, a condition is created in which the opening method differs for each product, confusing the user or requiring more time for fitting, and this may create a burden for the user.

In addition, when such a semi-processed continuous body is folded, using the method for producing a disposable wearable article of Patent Literature 2, the protrusion sections are folded in the first folding step and the side section regions are folded in the second folding step, which allows the aforementioned problem of stable folding to be solved. However, because two locations are folded at each side in the widthwise direction of the semi-processed product, the amount of preliminary manipulation during use increases and the time to fitting is increased, when the folded portions are to be opened during use of the absorbent article, thereby potentially increasing the burden of operation for the user.

It is an object of the invention is to provide a method for producing an absorbent article that allows the protrusion sections to be stably folded together with the side section regions, without increasing the burden of operation during use.

### Solution to Problem

A method for producing an absorbent article according to the present invention is as follows.
(1) A method for producing an absorbent article, comprising: a providing step of providing a semi-processed continuous body comprising semi-processed products for the absorbent article connected in a machine direction, the semi-processed product comprising a pair of protrusion sections formed protruding outward at both ends in the widthwise direction of the semi-processed product, and an expandable-and-shrinkable member that is expandable and shrinkable in an expansion-and-shrink direction and is disposed in an expanded state between the pair of protrusion sections so that the expansion-and-shrink direction is along the widthwise direction, a shrinking step of shrinking the expandable-and-shrinkable member to reduce a length in the widthwise direction at a portion including the expandable-and-shrinkable member in the semi-processed product to a length shorter than that before the shrinking, and form a plurality of wrinkles in the expandable-and-shrinkable member, a pressing step of pressing a center region in the widthwise direction of the semi-processed product, while transporting the semi-processed continuous body with the expandable-and-shrinkable member shrunk in the machine direction, and a folding step of raising, in a direction opposite from a direction of the pressing, a pair of side section regions on both outer sides in the widthwise direction of the center region of the semi-processed product at a pair of folding lines extending along the machine direction set more toward an inner side than both end sections in the widthwise direction of the expandable-and-shrinkable member, and folding each of the pair of side section regions upward above the center region, while transporting the semi-processed continuous body in the machine direction.

In this method for producing an absorbent article, the structure employs an expandable-and-shrinkable member (for example, a waist gather), to allow stable folding of the protrusion sections together with the side section regions, without increasing the burden of operation during use. In other words, since a plurality of wrinkles are formed in the region where the expandable-and-shrinkable member is disposed due to the function of the expandable-and-shrinkable member, at least some of the plurality of wrinkles are collapsed by the pressing member when the center region of the semi-processed product is pressed in the direction toward a mounting surface of the semi-processed product, due to the pressing member having a smaller length in the widthwise direction than the expandable-and-shrinkable member. As a result, the tops of the wrinkles that have convex shapes toward the side opposite the mounting surface side are pressed against the mounting surface side, and when the wrinkles are expanded, the pressed regions spread out in the widthwise direction on the mounting surface side. As the pressed regions spread out in the widthwise direction on the mounting surface side, the non-pressed regions, i.e., the regions further on the outer side in the widthwise direction than the pressed regions, instead rise up in the direction opposite the direction toward the mounting surface. Consequently, the portion of the semi-processed product including the expandable-and-shrinkable member has, throughout, concave shapes formed on the side opposite the mounting surface. As a result, the protrusion sections disposed on both sides in the widthwise direction of the side section regions also easily rise to follow the concave shapes. Furthermore, due to the function of the expandable-and-shrinkable member, the length in the widthwise direction at the portion of the semi-processed product including the expandable-and-shrinkable member becomes smaller than before shrink, or in other words, it becomes smaller than the length in the widthwise direction at the portion of the semi-processed product that does not include the expandable-and-shrinkable member, and therefore the protrusion sections approach the inner side in the widthwise direction of the absorbent article such that the protrusion sections approach the folding line. Consequently, the moment of inertia of the protrusion sections around the folding lines is reduced. This reduces the force necessary to rotate the protrusion sections with the folding lines as the center of rotation, or in other words, it reduces the force necessary to raise the protrusion sections. As a result it is possible for the protrusion sections to be easily raised. In this method for producing an absorbent article, by provision of the expandable-and-shrinkable member in the absorbent article (semi-processed product), at least the two functions described above act synergistically to allow the protrusion sections to be easily raised, thereby allowing the protrusion sections to be stably folded together with the side section regions. In this case, the portions that have been folded need only be opened at one location on each side during use of the absorbent article, and therefore the burden of operation for the user is not increased. In other words, it is possible to stably fold the protrusion sections together with the side section regions without increasing the burden of operation during use.

The method for producing an absorbent article according to the present invention may also be (2) the method for producing an absorbent article according to the above (1) wherein the folding lines are set so as to extend through the regions with the plurality of wrinkles.

By this method for producing an absorbent article, it is possible to easily fold the side section regions with the wrinkles as origins, in the folding step. This allows the protrusion sections on the outer sides in the widthwise direction of the side section regions to be stably raised and the protrusion sections to be more stably folded together with the side section regions.

The method for producing an absorbent article according to the present invention may also be (3) the method for producing an absorbent article according to the above (1) or (2) wherein the entire expandable-and-shrinkable member overlaps with the pair of protrusion sections in the machine direction.

By this method for producing an absorbent article, it is possible to more powerfully exhibit the effect of mounting the expandable-and-shrinkable member, or in other words, the effect of forming a concave shape in the expandable-and-shrinkable member by pressing of the wrinkles and the effect of lowering the moment of inertia of the protrusion sections around the folding lines.
This allows the protrusion sections on the outer sides in the widthwise direction of the side section regions to be stably raised and the protrusion sections to be more stably folded together with the side section regions.

The method for producing an absorbent article according to the present invention may also be (4) the method for producing an absorbent article according to any one of the above (1) to (3), wherein the semi-processed product comprises a pair of leak resistant walls extending along the machine direction and disposed separately in the widthwise direction, the leak resistant walls include rising sections which are folded so as to be capable of rising in a thickness direction of the semi-processed product, and in which edges on inner sides in the widthwise direction are free ends so as to be expandable and shrinkable and edges on outer sides in the widthwise direction are fixed ends, and the folding lines are set so as to extend through the rising sections.

Using this method for producing an absorbent article, it is possible to relatively increase the rigidity at the creases because the regions where the rising sections have been overlapped to have relatively higher rigidity overlap the folding lines. In other words, it can stabilize the creases. This allows the side section regions to be stably raised by the creases. In addition, it can aid in rising of the side section regions due to the shrink of the rising sections in the machine direction. This allows the protrusion sections on the outer sides in the widthwise direction of the side section regions to be more stably raised and the protrusion sections to be more stably folded together with the side section regions, in the same form.

The method for producing an absorbent article according to the present invention may also be (5) the method for producing an absorbent article according to the above (4), wherein the rising sections do not overlap the expandable-and-shrinkable member in the machine direction.

By this method for producing an absorbent article, expansion and shrink of the expandable-and-shrinkable member in the widthwise direction is not affected by expansion and shrink of the rising section in the machine direction. This allows the expandable-and-shrinkable member to be appropriately shrunk to form a plurality of wrinkles. Thus, it is possible to more powerfully exhibit the effect of mounting the expandable-and-shrinkable member, or in other words, the effect of forming a concave shape in the region of the semi-processed product including the expandable-and-shrinkable member and the effect of lowering the moment of inertia of the protrusion sections around the folding lines.
This allows the protrusion sections on the outer sides in the widthwise direction of the side section regions to be more stably raised and the protrusion sections to be more stably folded together with the side section regions.

The method for producing an absorbent article according to the present invention may also be (6) the method for producing an absorbent article according to the above (4) or (5), wherein the leak resistant walls comprise joint sections extending in the machine direction, connecting the rising sections to the semi-processed product at both ends in the machine direction of the rising sections, and the folding lines are set so as to extend through the joint sections.

Using this method for producing an absorbent article, it is possible to relatively increase the rigidity at the creases because the regions where the joint sections have been overlapped to have relatively higher rigidity overlap the folding lines. In other words, it can stabilize the creases. This allows the side section regions to be stably raised at the creases, and allows the protrusion sections on the outer sides in the widthwise direction of the side section regions to be more stably raised and the protrusion sections to be more stably folded together with the side section regions.

The method for producing an absorbent article according to the present invention may also be (7) the method for producing an absorbent article according to any one of the above (1) to (6), wherein the semi-processed product further comprises: another pair of protrusion sections disposed at both ends in the widthwise direction protruding toward the outer sides in the widthwise direction, and a to-be-connected member disposed between the other pair of protrusion sections, that is a member to which the pair of protrusion sections are joined during use of the absorbent article, and the folding lines are set so as to extend through the to-be-connected members.

Using this method for producing an absorbent article, it is possible to relatively increase the rigidity at the creases because the regions where the to-be-connected members have been overlapped to have relatively higher rigidity overlap the folding lines. In other words, it can stabilize the creases. This allows the other side section regions to be stably raised at the creases, and allows the other protrusion sections on the outer sides in the widthwise direction of the other side section regions to also be stably raised and the other protrusion sections to be more stably folded together with the side section regions.

The method for producing an absorbent article according to the present invention may also be (8) the method for producing an absorbent article according to any one of the above (1) to (7), wherein inner side edges of the pair of protrusion sections in the widthwise direction and outer side edges of the expandable-and-shrinkable member in the widthwise direction are mutually overlapping.

In this method for producing an absorbent article, the rigidity at the portions from the expandable-and-shrinkable member to the protrusion sections is relatively increased. Thus, with rising of the side section regions, the protrusion sections on the outer sides in the widthwise direction of the side section regions can be stably raised and the protrusion sections can be more stably folded together with the side section regions.

The method for producing an absorbent article according to the present invention may also be (9) the method for producing an absorbent article according to any one of the above (1) to (8), wherein the semi-processed product further comprises: an absorbent body disposed extending through a center in the widthwise direction and along the machine direction, and the folding lines are set so as to extend through edges in the widthwise direction of the absorbent body.

Using this method for producing an absorbent article, it is possible to relatively increase the rigidity at the creases because the regions where the absorbent body in the semi-processed product has been overlapped to have relatively higher rigidity overlaps the folding lines. In other words, it can stabilize the creases. This allows the side section regions to be stably raised at the creases, and allows the protrusion sections disposed on the outer sides in the widthwise direction of the side section regions to be stably raised so that the protrusion sections can be more stably folded together with the side section regions.

### Advantageous Effects of Invention

The present invention can provide a method for producing an absorbent article that allows the protrusion sections to be stably folded together with the side section regions, without increasing the burden of operation during use.

### Brief Description of Drawings

Fig. 1 is a diagram showing an absorbent article according to an embodiment.
Fig. 2 is a diagram showing an absorbent article according to the embodiment.
Fig. 3 is a schematic overview of a production apparatus for an absorbent article according to the embodiment.
Fig. 4 is a schematic overview of a widening mechanism for an absorbent article according to the embodiment.
Fig. 5 is a schematic overview of a folding apparatus for an absorbent article according to the embodiment.
Fig. 6 is a diagram illustrating a method for producing an absorbent article according to the embodiment.
Fig. 7 is a diagram illustrating a method for producing an absorbent article according to the embodiment.
Fig. 8 is a diagram illustrating a method of folding an absorbent article according to the embodiment.
Fig. 9 is a diagram illustrating a method of folding an absorbent article according to the embodiment.
Fig. 10 is a diagram illustrating a method for producing an absorbent article according to the embodiment.
Fig. 11 is a diagram illustrating a method for producing an absorbent article according to the embodiment.

### Description of Embodiments

An absorbent article according to the embodiment will now be explained with reference to the attached drawings, using a tape-type (open-type) disposable diaper as an example. However, the types and uses of the absorbent article of the present invention are not limited to this example, and the present invention may be applied to other absorbent articles such as panty-type disposable diapers, for example, without departing from the scope of the subject matter of the present invention.

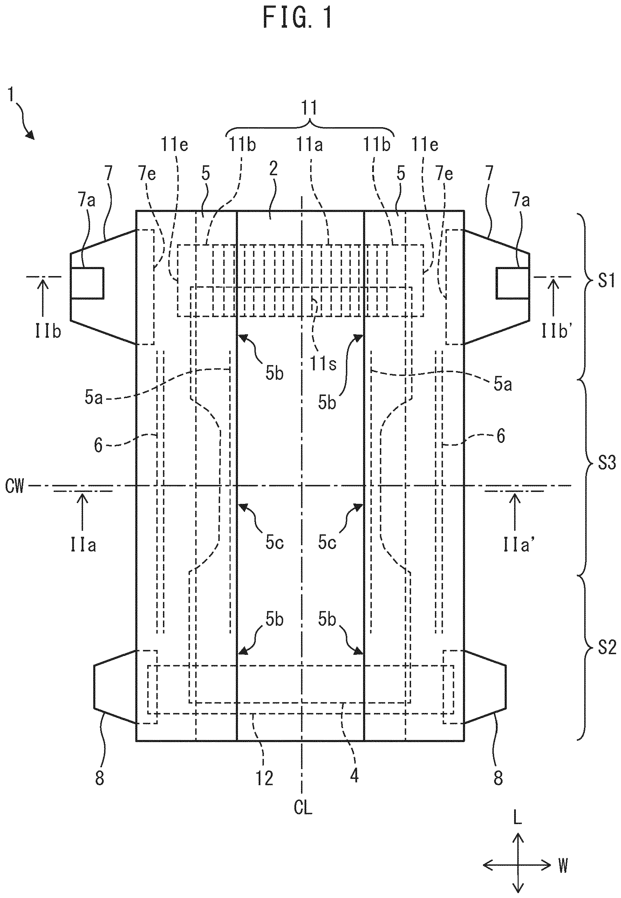

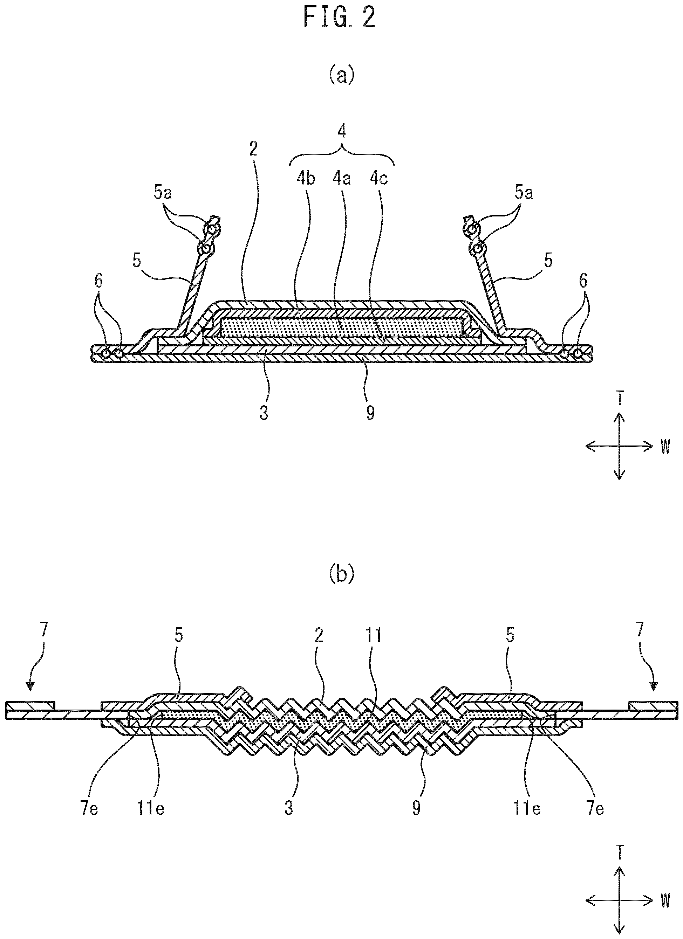

Fig. 1 and Fig. 2 are diagrams showing an absorbent article 1 (disposable diaper), and specifically, Fig. 1 shows a plan view of the absorbent article 1 in the deployed, spread-out state, Fig. 2(a) is a cross-sectional view along line IIa-IIa' of Fig. 1, and Fig. 2(b) is a cross-sectional view along line IIb-IIb' of Fig. 1. However, Fig. 2(b) shows the absorbent article in the natural state, i.e., the state in which the expandable-and-shrinkable member 11 is shrunk, as described later. The absorbent article 1 has a lengthwise direction L, a widthwise direction W perpendicular to the lengthwise direction L, and a thickness direction T perpendicular to the lengthwise direction L and the widthwise direction W, and also has a center axis line CL extending through the center of the widthwise direction W and extending in the lengthwise direction L, and a center axis line CW extending through the center of the lengthwise direction L and extending in the widthwise direction W.

The absorbent article 1 also has, in the lengthwise direction L, a back girth region S1 corresponding to the girth around the back side of the wearer, an abdominal girth region S2 corresponding to the girth around the abdominal side of the wearer, and a groin region S3 located between the back girth region S1 and the abdominal girth region S2, corresponding to the groin of the wearer. It is worn as a diaper by connecting both ends in the widthwise direction W of the back girth region S1 and both ends in the widthwise direction W of the abdominal girth region S2. The groin region S3 may be narrowed in the widthwise direction W.

Throughout the present description, unless otherwise specified, the simple phrase "as plane view" will be used to mean that the absorbent article 1 in the deployed spread-out flat state is viewed from the upper side in the thickness direction. The terms "skin side" and "non-skin side" refer, respectively, to the side relatively near the skin side and the side relatively away from the skin side of the wearer, in the thickness direction of the absorbent article, when the wearer of the absorbent article has worn the absorbent article. Also, the direction toward the center axis line CL is the direction on the inner side in the widthwise direction W, and the direction receding from the center axis line CL is the direction on the outer side in the widthwise direction W. The direction toward the center axis line CW is the direction on the inner side in the lengthwise direction L, and the direction receding from the center axis line CW is the direction on the outer side in the lengthwise direction L.

The absorbent article 1 comprises a front sheet 2, a back sheet 3 and an absorbent body 4. However, the lengthwise direction, widthwise direction and thickness direction of the front sheet 2, back sheet 3 and absorbent body 4 all match the lengthwise direction L, widthwise direction W and thickness direction T of the absorbent article 1. Thus, the lengthwise direction L, widthwise direction W and thickness direction T are each used for the lengthwise direction, widthwise direction and thickness direction for the front sheet 2, back sheet 3 and absorbent body 4 as well, and the skin side and non-skin side are respectively used for the side relatively near and the side relatively away from the skin side of the wearer, in the thickness direction.

The front sheet 2 is a liquid-permeable sheet situated on the skin side of the wearer. The front sheet 2 may be any desired liquid-permeable sheet, such as, for example, a liquid-permeable nonwoven fabric or woven fabric, a liquid-permeable porous synthetic resin film, or a composite sheet thereof. The back sheet 3 is a liquid-impermeable sheet situated on the non-skin side of the wearer. The back sheet 3 may be any desired liquid-impermeable sheet, such as, for example, a liquid-impermeable nonwoven fabric or synthetic resin film, a composite sheet thereof, an SMS nonwoven fabric, or the like. The absorbent body 4 is a fluid-absorbing and liquid-retaining material situated between the front sheet 2 and the back sheet 3, and for this embodiment, it includes the absorbent body core 4a and core wraps 4b, 4c that subsume the absorbent body core 4a. The absorbent body 4 may be pulp fiber, synthetic fiber, an absorbing polymer or the like. The absorbent body 4 is bonded to each of the front sheet 2 and back sheet 3 with an adhesive, the front sheet 2 and back sheet 3 being bonded by an adhesive at their peripheries. The adhesive used for bonding between the front sheet 2, absorbent body 4 and back sheet 3 may be a known material that is commonly used in absorbent articles 1, such as a thermoplastic adhesive.

The absorbent article 1 further comprises a pair of leak resistant walls 5, 5, leg expandable-and-shrinkable members 6, 6 and an outer sheet 9. The pair of leak resistant walls 5, 5 are a pair of side section sheets that extend along the lengthwise direction L so as to cover the surfaces of both sides in the widthwise direction W of the front sheet 2, and they are disposed across a distance in the widthwise direction W. The pair of leak resistant walls 5, 5 have their fixed ends at the edges on the outer sides in the widthwise direction W fixed on the surface of both sides in the widthwise direction W of the front sheet 2, and have their free ends forming gather sections that are expandable and shrinkable at the edges on the inner side in the widthwise direction W, also including folded rising sections 5c that are able to rise up in the thickness direction T. Near each of the free ends of the rising sections 5c, 5c of the pair of leak resistant walls 5, 5, there are situated two linear elastic solids 5a such as rubber, for example, extending along the lengthwise direction L. Each elastic solid 5a shrinks the free end of the leak resistant wall 5, thereby causing the pair of leak resistant walls 5, 5 to expand and rise. The pair of leak resistant walls 5, 5 each further include, at both ends in the lengthwise direction L of the rising section 5c, joint sections 5b extending in the lengthwise direction L, where the rising section 5c is connected to the front sheet 2. The leg expandable-and-shrinkable member 6 is an elastic material such as rubber that expands in the lengthwise direction L of each of both sides in the widthwise direction W of the groin region S3 that contacts with the femoral region of the wearer. The outer sheet 9 reinforces the back sheet 3 on the non-skin side of the back sheet 3, and improves its hand feel. It is mutually connected with the pair of leak resistant walls 5, 5 at the peripheral sections while being joined to the non-skin side of the back sheet 3. There are no particular restrictions on the materials for the leak resistant walls 5 and the outer sheet 9, and examples include the material for the front sheet 2, for the leak resistant walls 5, and the material for the back sheet 3, for the outer sheet 9.

The absorbent article 1 further comprises an expandable-and-shrinkable member 11 and a pair of protrusion sections 7 in the back girth region S1. The expandable-and-shrinkable member 11 is a sheet that is expandable and shrinkable in the expansion-and-shrink direction, and is disposed between the pair of protrusion sections 7, 7 so as to be along the expansion-and-shrink direction in the widthwise direction W, i.e., expandable and shrinkable in the widthwise direction W, functioning as a waist gather, for example. The expandable-and-shrinkable member 11 is attached using an adhesive to any location of the surface on the skin side of the front sheet 2, the surface on the non-skin side of the back sheet 3, and the region between the front sheet 2 and the back sheet 3, in the back girth region S1. The expandable-and-shrinkable member 11 is also disposed either partially overlapping or without any overlapping with the absorbent body 4, as plane view. For this embodiment, the expandable-and-shrinkable member 11 is disposed between the front sheet 2 and the back sheet 3 in such a manner that the portion on the inner side in the lengthwise direction L of the expandable-and-shrinkable member 11 overlaps with the absorbent body 4 as plane view.

The expandable-and-shrinkable member 11 also includes a high-expanded region 11a located at the center in the widthwise direction W and low-expanded regions 11b located at both sides in the widthwise direction W of the high-expanded region 11a. The high-expanded region 11a is attached to the front sheet 2 and/or back sheet 3 with the expandable-and-shrinkable member 11 in a widely expanded state, and it undergoes large shrink together with the front sheet 2 and/or back sheet 3 by returning to the natural state after having been expanded, whereby a plurality of wrinkles 11S are formed. The wrinkles 11S extend generally in the lengthwise direction L and are arranged generally in the widthwise direction W. The low-expanded regions 11b are attached to the front sheet 2 and/or back sheet 3 with the expandable-and-shrinkable member 11 in a modestly expanded state, and they shrink moderately together with the front sheet 2 and/or back sheet 3 by returning to the natural state after having been expanded, such that few wrinkles are formed. Thus, the high-expanded region 11a is a region capable of high expansion and shrink, and the low-expanded regions 11b are regions capable of low expansion and shrink.
However, the low-expanded regions 11b are not essential, and the entirety may consist of the high-expanded region 11a. The material of the expandable-and-shrinkable member 11 is not particularly restricted so long as it is expandable and shrinkable, and it may be expandable and shrinkable as a material, or expandable and shrinkable in shape, or expandable and shrinkable by combination with an elastic member.

The pair of protrusion sections 7, 7 are sheets for connection of the back girth region S1 to the abdominal girth region S2 during wear, and they function as side flaps. The pair of protrusion sections 7, 7 are disposed so as to protrude toward both outer sides in the widthwise direction W of the back girth region S1, and are attached with an adhesive at any location on the surface of the skin side of the front sheet 2 (or leak resistant wall 5), on the surface of the non-skin side of the back sheet 3 (or outer sheet 9), in the region between the front sheet 2 (or leak resistant wall 5) and the back sheet 3 (or outer sheet 9) in the back girth region S1. However, the pair of protrusion sections 7, 7 may also be formed at the extended sections of either or both the front sheet 2 (or leak resistant wall 5) and the back sheet 3 (or outer sheet 9). For this embodiment, the protrusion sections 7 are disposed between the leak resistant wall 5 and the outer sheet 9, with separate members from the front sheet 2, leak resistant wall 5, back sheet 3 and outer sheet 9. The protrusion sections 7 comprise connecting tapes 7a for connection with a to-be-connected member 12 (described below) in the abdominal girth region S2 during wear. The connecting tapes 7a may be a hook-and-loop fastener or adhesive tape. The material for the protrusion sections 7 is not particularly restricted, and may be the material of the front sheet 2 or back sheet 3, for example.

The expandable-and-shrinkable member 11 is disposed between the pair of protrusion sections 7, 7. That is, it is disposed in such a manner that the expandable-and-shrinkable member 11 and the pair of protrusion sections 7, 7 overlap in at least sections in the lengthwise direction L. Stated differently, projection onto the center axis line CL of the edge line 11e along the lengthwise direction L on the outer side in the widthwise direction W of the expandable-and-shrinkable member 11, and projection onto the center axis line CL of the edge line 7e along the lengthwise direction L on the inner side in the widthwise direction W of the protrusion section 7, overlap at least partially. This can improve the fitting property of the absorbent article 1 in the girth region during wear.

The absorbent article 1 further comprises a to-be-connected member 12 and a pair of protrusion sections 8 in the abdominal girth region S2. The to-be-connected member 12 is a sheet that is to be connected with the connecting tapes 7a of the pair of protrusion sections 7, 7, and when the connecting tapes 7a are hooks of hook-and-loop fasteners, for example, the to-be-connected member 12 is a loop of a hook-and-loop fastener, and when the connecting tapes 7a are adhesive tapes, the to-be-connected member 12 is a sheet capable of pressure-sensitive adhesion with the adhesive tape. The to-be-connected member 12 is attached with an adhesive at a location on the surface on the non-skin side of the outer sheet 9 in the abdominal girth region S2. The to-be-connected member 12 is also disposed either partially overlapping or without any overlapping with the absorbent body 4, as plane view. For this embodiment, the to-be-connected member 12 is disposed on the surface on the non-skin side of the outer sheet 9, in such a manner that a portion on the inner side in the lengthwise direction L of the to-be-connected member 12 overlaps with the absorbent body 4, as plane view. The pair of protrusion sections 8, 8 are sheets for connection of the back girth region S2 to the abdominal girth region S1 during wear, and they function as side flaps. The pair of protrusion sections 8 are disposed so as to protrude toward both outer sides in the widthwise direction W of the abdominal girth region S2, and are attached with an adhesive at any location on the surface of the skin side of the front sheet 2 (or leak resistant wall 5), on the surface of the non-skin side of the back sheet 3 (or outer sheet 9), and in the region between the front sheet 2 (or leak resistant wall 5) and the back sheet 3 (or outer sheet 9) in the abdominal girth region S2. However, the pair of protrusion sections 8, 8 may also be formed at the extended sections of either or both the front sheet 2 (or leak resistant wall 5) and the back sheet 3 (or outer sheet 9). For this embodiment, the protrusion sections 8 are disposed between the leak resistant wall 5 and the outer sheet 9, with separate members from the front sheet 2, leak resistant wall 5, back sheet 3 and outer sheet 9. The material for the protrusion sections 8 is not particularly restricted, and may be the material of the front sheet 2 or back sheet 3, for example. The protrusion sections 8 may also be absent.

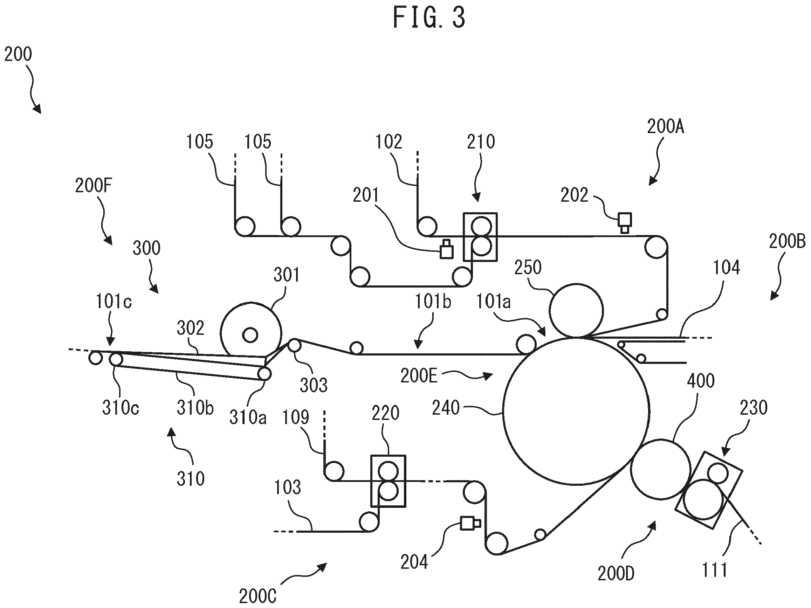

A production apparatus for an absorbent article according to this embodiment will now be described. Fig. 3 shows an example of a configuration of a production apparatus 200 for an absorbent article 1. The production apparatus 200 comprises a front sheet-forming unit 200A, an absorbent body-forming unit 200B, a back sheet-forming unit 200C, an expandable-and-shrinkable member-bonding unit 200D, a bonding unit 200E and a folding unit 200F.

For transport of the absorbent article 1 and the materials composing it, the production apparatus 200 has a machine direction MD, a cross-machine direction CD perpendicular to the machine direction MD along the transport surface, and a thickness direction TD perpendicular to the machine direction MD and the cross-machine direction CD. However, the lengthwise direction, widthwise direction and thickness direction of the absorbent article 1 and the materials composing it are the same as the machine direction MD, cross-machine direction CD and thickness direction TD, respectively. Thus, for the absorbent article 1 and the materials composing it as well, the machine direction MD, cross-machine direction CD and thickness direction TD are used as the lengthwise direction, widthwise direction and thickness direction, respectively.

In the front sheet-forming unit 200A, a plurality of transport rolls convey a continuous front sheet 102, as a continuous sheet for the front sheet, to a bonding roll 210. An adhesive coating applicator 201 coats the adhesive onto one side of the continuous front sheet 102 during transport. Separately, a plurality of transport rolls convey a pair of continuous leak resistant wall sheets 105, 105, as continuous sheets for the pairs of leak resistant walls, to the bonding roll 210. The bonding roll 210 compresses the continuous front sheet 102 and the pair of continuous leak resistant wall sheets 105, 105 sandwiched between the pair of bonding rolls that are disposed mutually facing each other, forming a continuous front sheet 102 with the pair of continuous leak resistant wall sheets 105, 105 bonded with an adhesive on both outer sides in the cross-machine direction. The adhesive coating applicator 202 coats the adhesive on the other side, which is the side of the transported continuous front sheet 102 opposite the side on which the continuous leak resistant wall sheets 105, 105 are bonded. A plurality of transport rolls supply the continuous front sheet 102 to the bonding unit 200E (bonding rolls 240, 250).

At the absorbent body-forming unit 200B, the conveyor belt supplies an absorbent body 104 formed by an absorbent body-forming apparatus (not shown) to the bonding unit 200E (bonding rolls 240, 250).

In the back sheet-forming unit 200C, a plurality of transport rolls convey a continuous back sheet 103, as a continuous sheet for the back sheet, to a bonding roll 220. Separately, a plurality of transport rolls transport a continuous outer sheet 109 (with the leg expandable-and-shrinkable member 6, protrusion sections 7, 8 and to-be-connected member 12 already added) as a continuous sheet for the outer sheet, to the bonding roll 220. An adhesive coating applicator 203 coats an adhesive onto one side of the continuous outer sheet 109 during transport. The bonding roll 220 compresses the continuous outer sheet 109 and the continuous back sheet 103 sandwiched between the pair of bonding rolls that are disposed mutually facing each other, forming a continuous back sheet 103 with the continuous outer sheet 109 bonded on the back side. An adhesive coating applicator 204 coats the adhesive on the other side of the transported continuous back sheet 103 where the continuous outer sheet 109 is not bonded. A plurality of transport rolls supply the continuous back sheet 103 to the bonding unit 200E (bonding roll 240).

In the expandable-and-shrinkable member-bonding unit 200D, a plurality of transport rolls transport a continuous expandable-and-shrinkable member sheet 111, which is a continuous sheet for the expandable-and-shrinkable members, to a cutting apparatus 230. The cutting apparatus 230 cuts the continuous expandable-and-shrinkable member sheet 111 into prescribed lengths with a cutter. A widening/attaching apparatus 400 receives the cut fragments, i.e., the expandable-and-shrinkable member 11, while holding both end sections of the expandable-and-shrinkable member in the cross-machine direction. The widening/attaching apparatus 400 expands the expandable-and-shrinkable member 11 in the cross-machine direction while rotating approximately 180°. Next, the widening/attaching apparatus 400 presses the expandable-and-shrinkable member 11 in the expanded state against the side of the continuous back sheet 103 on which the adhesive was coated, which was on the bonding roll 240, to attach it to the continuous back sheet 103. Since the continuous back sheet 103 is under tension in the machine direction MD during this time, the continuous back sheet 103 undergoes virtually no narrowing even when the expandable-and-shrinkable member 11 in the extended expanded out state in the cross-machine direction CD is attached. The bonding roll 240 moves the continuous back sheet 103 onto which the expandable-and-shrinkable member 11 has been attached, to the bonding unit 200E (bonding roll 250).

At the bonding unit 200E, the pair of bonding rolls 240, 250 that are disposed mutually facing each other compress the continuous front sheet 102 from the front sheet-forming unit 200A, the absorbent body 104 from the absorbent body-forming unit 200B and the continuous back sheet 103 from the back sheet-forming unit 200C while sandwiching them, to form a semi-processed continuous body 101a of a semi-processed absorbent article having the continuous front sheet 102, the absorbent body 104 and the continuous back sheet 103 laminated.

Next, a plurality of transport rolls transport the semi-processed continuous body 101a away from the bonding roll 240, while reducing the tension in the machine direction MD. This forms a semi-processed continuous body 101b in which the portion where the expandable-and-shrinkable member is disposed is somewhat shrunk in the cross-machine direction CD. A plurality of transport rolls transport the semi-processed continuous body 101b to the folding unit 200F.

At the folding unit 200F, a folding apparatus 300 transports the semi-processed continuous body 101b by a transport roll 303 and a conveying apparatus 310, while folding it with a pressing member 301 and a folding member 302, to form a semi-processed continuous body 101c. The semi-processed continuous body 101c is then cut into individual absorbent article product portions as the absorbent article 1.

The widening/attaching apparatus 400 of the expandable-and-shrinkable member-bonding unit 200D will now be explained.

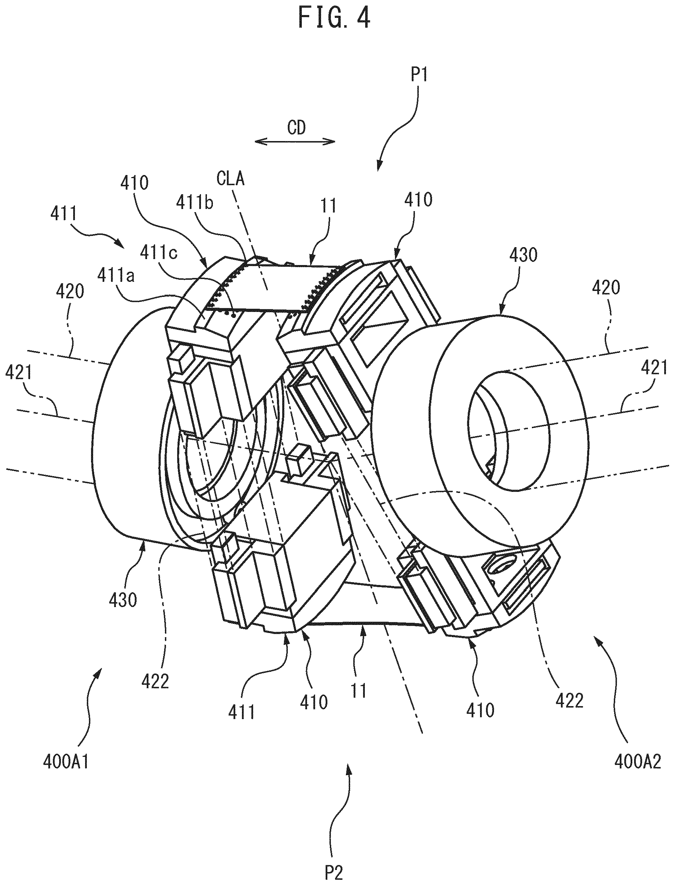

Fig. 4 is a perspective view schematically showing an example of a configuration of the widening/attaching apparatus 400. The widening/attaching apparatus 400 is an apparatus that receives a belt-shaped width-shrunk member 405 at a receiving position P1 and expands it in the cross-machine direction CD while delivering it to the continuous back sheet 103 at a delivering position P2. The widening/attaching apparatus 400 comprises a pair of widening units 400A1, 400A2 having bilateral symmetry with respect to the center line CLA. Each widening unit 400A comprises a relative moving member 430, a transport driving member 420 and a pair of transporting members 410, 410. The pair of widening units 400A1, 400A2 are set in positions so as to be mutually close at one end and mutually far at the other end (they are divergent with respect to each other). In this case, the receiving position P1 is where they are mutually close and the delivering position P2 is where they are mutually far.

The transport driving member 420 has a rotor 422 that rotates around a rotating shaft 421 as the center, the rotor 422 being mounted in a freely rotatable manner around the relative moving member 430 via a bearing. In the rotor 422, the pair of transporting members 410 are anchored at positions mutually separated 180° in the rotational direction of the rotor 422. Consequently, by rotation of the pair of transport driving members 420 at the same angular speed, rotation takes place around the relative moving member 430, with the transport members 410 facing each other on the rotors 422 of both widening units 400A1, 400A2. Each of the transport members 410 comprises a suction pad 411. The suction pad 411 is situated on the outer side surface of the transport member 410, and has a contact surface 411a with which the expandable-and-shrinkable member 11 contacts. A plurality of holes are formed in the contact surface 411a, some of the holes being suction holes 411c for suctioning of the expandable-and-shrinkable member 11 in contact with the contact surface 411a, by a suction mechanism (not shown) situated in the transport member 410, while the other holes are pinholes 411b through which pin members are inserted to anchor the expandable-and-shrinkable member 11 in contact with the contact surface 411a.

At the receiving position P1, the widening/attaching apparatus 400 receives the expandable-and-shrinkable member 11 by suctioning both end sections of the expandable-and-shrinkable member 11 by the suction holes 411c of the pair of transporting members 410. At the same time, the pin members of the pinholes 411b pierce the expandable-and-shrinkable member 11, thereby holding the expandable-and-shrinkable member 11 by the pair of transporting members 410. The widening/attaching apparatus 400 rotationally moves the pair of transporting members 410 by the pair of transport driving members 420, causing the expandable-and-shrinkable member 11 to move from the receiving position P1 to the delivering position P2. At this time, the pair of transporting members 410 rotationally move while gradually widening the distance between the suction pads 411, such that both end sections of the expandable-and-shrinkable member 11 are pulled and expanded. Also, the widening/attaching apparatus 400 delivers the expandable-and-shrinkable member 11 by termination of the suction and pin piercing with the pair of transporting members 410 at the delivering position P2.

By adjusting the widening units 400A1, 400A2 at the widening/attaching apparatus 400, for example, the center section in the cross-machine direction CD of the expandable-and-shrinkable member 11 (for example, the portion on the inner side between the suction pads 411) can be the region with high expanding (high-expanded region) and the both end sections on both sides thereof (for example, the portions of the suction pads 411) can be the regions with low expanding (low-expanded regions), or the entire expandable-and-shrinkable member 11 can be the high-expanded region. Adjustment of the widening units 400A1, 400A2 may be, for example, adjustment of the distance between the widening units 400A1, 400A2, the degree of suction of the suction pads 411, or the presence or absence of insertion of the pin members in the pinholes 411b.

The folding apparatus 300 of the folding unit 200F will now be explained.

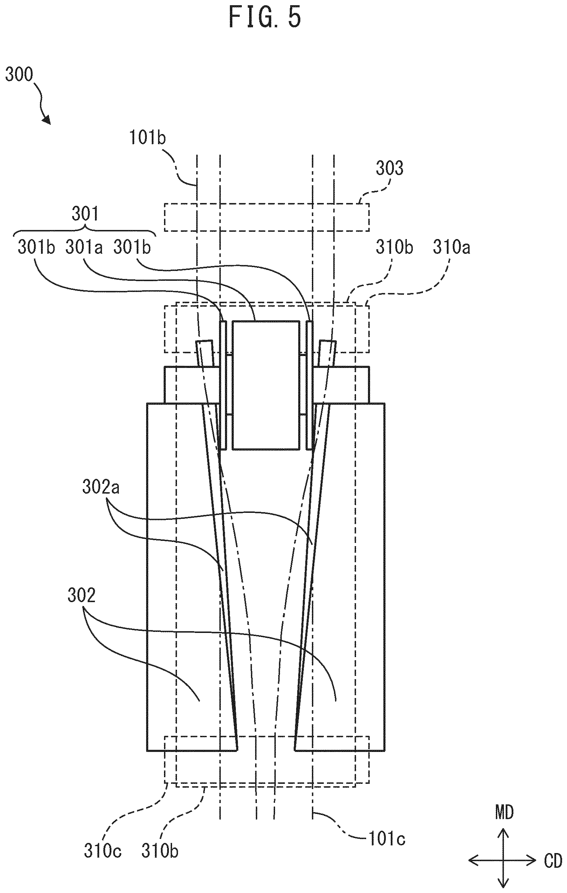

Fig. 5 is a diagram schematically showing an example of a configuration of the folding apparatus 300. The folding apparatus 300 is an apparatus that folds a continuous body at a prescribed folding position while transporting the continuous body in the machine direction MD, and it comprises a transport roll 303, a conveying apparatus 310, a pressing member 301 and a folding member 302. The semi-processed continuous body 101b will be used as an example of the continuous body.

The transport roll 303 supplies the semi-processed continuous body 101b to the pressing member 301 and the conveying apparatus 310, located downstream in the machine direction. The pressing member 301 is disposed on the conveying apparatus 310, downstream form the transport roll 303 in the machine direction MD. In the cross-machine direction CD, the length of the pressing member 301 is set to be narrower than the length of the semi-processed continuous body 101b. The pressing member 301 presses at least the center region of the semi-processed continuous body 101b toward the direction crossing the surface of the semi-processed continuous body 101b, or in other words, toward the surface of the conveying apparatus 310 on which the semi-processed continuous body 101b is mounted, while transporting the semi-processed continuous body 101b in the machine direction. The conveying apparatus 310 rotates an endless belt 310b in the machine direction MD by a roller 310a or a roller 310c, transporting the semi-processed continuous body 101b in the machine direction MD. A suction mechanism is mounted in the conveying apparatus 310, and the center region of the semi-processed continuous body 101b is pressed toward the endless belt 310b by the pressing member 301, while being suctioned to the transport surface of the endless belt 310b (the mounting surface of the semi-processed continuous body 101b). The folding member 302 guides the side section regions on both sides in the cross-machine direction CD of the semi-processed continuous body 101b that is being transported along the machine direction MD, onto the center region, by guides 302a, 302a. The suction mechanism does not need to be mounted on the conveying apparatus 310. The pressing member 301 has a pair of presser rolls 301b, 301b that press the semi-processed continuous body 101b, and a center roll 301a that is disposed between the presser rolls 301b, 301b and presses the center region of the semi-processed continuous body 101b. The position of the semi-processed continuous body 101b pressed by the presser rolls 301b, 301b becomes the folding origin, and the line drawn along the machine direction MD at that position becomes the folding line. The presser rolls 301b, 301b can be moved to any desired position between both ends and the center of the semi-processed continuous body 101b in the cross-machine direction CD. Thus, the folding line can be set with the folding apparatus 300 along the machine direction MD, at any desired position in the cross-machine direction CD. The presser rolls 301b, 301b may even be absent, in which case the function of the presser rolls 301b, 301b is carried out by both ends of the center roll 301a in the cross-machine direction CD.

At the folding apparatus 300, the transport roll 303 is disposed at a position of height h higher than the transport surface of the endless belt 310b, the width of the pressing member 301 being smaller than the width of the semi-processed continuous body 101b. This causes upward turning force to act from the transport surface of the endless belt 310b onto the pressing member 301 sides, in the side section regions of the semi-processed continuous body 101b that are not pressed against the pressing member 301. The upwardly turned side section regions are guided by the guides 302a, 302a, and fall back onto the center region. This allows folding of the side section regions (the regions connected in the machine direction MD).

For this embodiment, the width (length) of the pressing member 301 in the cross-machine direction CD is set to be narrower than the width (length) of the semi-processed continuous body 101b and narrower than the width (length) of the expandable-and-shrinkable member 11. It is preferably set to be narrower than the width (length) of the high-expanded region 11a of the expandable-and-shrinkable member 11. This allows the semi-processed continuous body 101b to be easily folded, as described below. The semi-processed continuous body 101b is folded in the folding apparatus 300 as a semi-processed continuous body 101c. The semi-processed continuous body 101c is then cut into individual absorbent article product portions as the absorbent article 1.

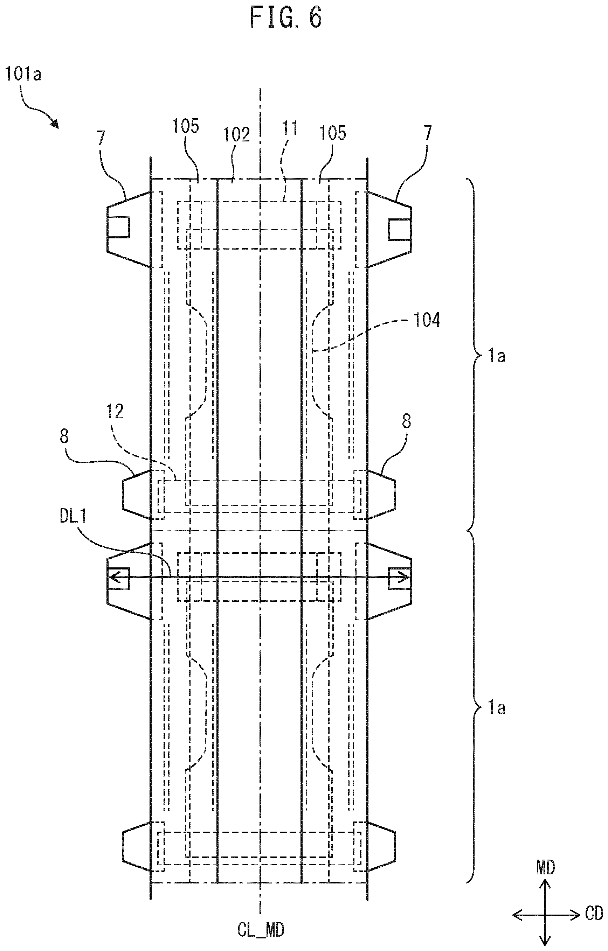

A method for producing the absorbent article of this embodiment will now be explained with reference to Fig. 3 to Fig. 5 and Fig. 6 to Fig. 11. Fig. 6 to Fig. 11 schematically show an example of the configuration of a semi-processed product, for explanation of the method for producing an absorbent article. In Fig. 6 to Fig. 11, the product runs from top to bottom in the drawing, in the machine direction MD. The production method is a method for producing an absorbent article, and it comprises a front sheet-forming step, an absorbent body-forming step, a back sheet-forming step, an expandable-and-shrinkable member bonding step, a bonding step (the above steps being the providing step), a shrinking step, a pressing step and a folding step.

As shown in Fig. 3, in the front sheet-forming step, a continuous front sheet 102 which is the continuous sheet for the front sheet is coated with an adhesive on one side by the adhesive coating applicator 201, and then supplied to the bonding roll 210. Meanwhile, a pair of continuous leak resistant wall sheets 105, 105 as continuous sheets for the pairs of leak resistant walls, are supplied to the bonding roll 210. At the bonding roll 210, the continuous front sheet 102 and the pair of continuous leak resistant wall sheets 105, 105 are supplied between the pair of bonding rolls. The pair of continuous leak resistant wall sheets 105, 105 are pressed against the side of the continuous front sheet 102 on which the adhesive has been coated, thereby forming a continuous front sheet 102 having the pair of continuous leak resistant wall sheets 105, 105 bonded to both outer sides in the cross-machine direction. Next, by the adhesive coating applicator 202, the continuous front sheet 102 is coated with an adhesive on the other side which is the side opposite the side on which the continuous leak resistant wall sheets 105, 105 have been bonded, and then supplied to the bonding unit 200E (bonding rolls 240, 250).

In the absorbent body-forming step, an absorbent body 104 is formed at an absorbent body-forming apparatus (not shown), and supplied to the bonding unit 200E (bonding rolls 240, 250) by a conveyor belt.

In the back sheet-forming step, a continuous back sheet 103 which is the continuous sheet for the back sheet is supplied to the bonding roll 220. Meanwhile, a continuous outer sheet 109 (with the leg expandable-and-shrinkable member 6, protrusion sections 7, 8 and to-be-connected member 12 added), which is the continuous sheet for the outer sheet, is coated with an adhesive on one side by the adhesive coating applicator 203, and supplied to the bonding roll 220. At the bonding roll 220, the continuous outer sheet 109 and the continuous back sheet 103 are supplied between a pair of bonding rolls. Also, the continuous back sheet 103 is pressed against the side of the continuous outer sheet 109 on which the adhesive has been coated to attach them both, thereby forming a continuous back sheet 103 in which the continuous outer sheet 109 is bonded to the back side. Next, by the adhesive coating applicator 204, the continuous back sheet 103 is coated with an adhesive on the other side on which the continuous outer sheet 109 is not bonded, and then supplied to the bonding unit 200E (bonding roll 240) .

In the expandable-and-shrinkable member bonding step, a continuous expandable-and-shrinkable member sheet 111 which is the continuous sheet for the expandable-and-shrinkable members, is supplied to the cutting apparatus 230, and the tip sections in the machine direction MD are cut to a prescribed length by the cutter of the cutting apparatus 230. Each cut fragment, i.e., the expandable-and-shrinkable member 11, is held at the both end sections in the cross-machine direction CD by the widening/attaching apparatus 400, and received by the widening/attaching apparatus 400. The expandable-and-shrinkable member 11 is rotated approximately 180° while the both end sections in the cross-machine direction CD are expanded in the cross-machine direction by the widening/attaching apparatus 400, and is then pressed against the adhesive-coated surface of the continuous back sheet 103 on the bonding roll 240 in the expanded state, and is attached to the continuous back sheet 103. Since the continuous back sheet 103 is under tension in the machine direction during this time, the continuous back sheet 103 undergoes virtually no narrowing even when the expandable-and-shrinkable member 11 in the extended expanded out state in the cross-machine direction CD is attached. The continuous back sheet 103 onto which the expandable-and-shrinkable member 11 has been attached moves over the bonding roll 240 to the bonding unit 200E (bonding roll 250).

In the bonding step, the continuous front sheet 102 on which the continuous leak resistant wall sheets 105, 105 are bonded from the front sheet-forming step, the absorbent body 104 from the absorbent body-forming step, and the continuous back sheet 103 on which the expandable-and-shrinkable member 11 is attached from the expandable-and-shrinkable member bonding step, are transported between the pair of bonding rolls 240, 250. Also, the continuous front sheet 102, the absorbent body 104 and the continuous back sheet 103 are sandwiched, compressed and bonded between the pair of bonding rolls 240, 250. A semi-processed continuous body 101a of the absorbent article is thus formed comprising the continuous front sheet 102, the continuous back sheet 103, and the absorbent body 104 lying between the continuous front sheet 102 and the continuous back sheet 103. The front sheet-forming step, absorbent body-forming step, back sheet-forming step, expandable-and-shrinkable member bonding step and bonding step may be considered as an example of the providing step for providing the semi-processed continuous body 101a. However, the providing step is not limited to this example, and for example, it may be a step of providing a pre-formed semi-processed continuous body 101a.

Fig. 6 schematically shows an example of the structure of a semi-processed continuous body 101a. The semi-processed continuous body 101a is composed of a plurality of semi-processed products 1a connected in the machine direction. One semi-processed product 1a has virtually no wrinkles 11S in the expandable-and-shrinkable member 11, and has the same configuration as the absorbent article 1 shown in Fig. 1. That is, the semi-processed product 1a comprises a pair of protrusion sections 7, 7 formed protruding outward at both ends in the widthwise direction of the semi-processed product 1a, i.e., the cross-machine direction CD, and an expandable-and-shrinkable member 11 that is expandable and shrinkable in the expansion-and-shrink direction and is disposed in an expanded state between the pair of protrusion sections 7, 7 so that the expansion-and-shrink direction lies along the cross-machine direction CD. In the lengthwise direction, or machine direction MD, of the semi-processed product 1a, the expandable-and-shrinkable member 11 and protrusion section 7 are at least partially overlapping. However in the cross-machine direction CD of the semi-processed product 1a, the expandable-and-shrinkable member 11 and the protrusion sections 7 may be partially overlapping, or they may be non-overlapping.

In the cross-machine direction CD, the maximum length at the portion including the expandable-and-shrinkable member 11 of the semi-processed product 1a (including the lengths of the protruding sections of the pair of protrusion sections 7, 7) is DL1. In this case, the expandable-and-shrinkable member 11 of the semi-processed product 1a undergoes virtually no shrink by tension on the semi-processed continuous body 101a.
Thus, the expandable-and-shrinkable member 11 is disposed in the expanded state in the cross-machine direction CD, but is only slightly shrunk.

In the shrinking step, the semi-processed continuous body 101a separates from the bonding roll 240 and tension in the machine direction MD is reduced (for example, the rotational speed of the transport roll is reduced). This allows the expandable-and-shrinkable member 11 expanded in the cross-machine direction CD to shrink, thereby allowing the expandable-and-shrinkable member 11 to be shrunk in the cross-machine direction CD. In response, the portion including the expandable-and-shrinkable member 11 of the semi-processed product 1a can also be shrunk in the cross-machine direction CD, whereby a semi-processed continuous body 101b is formed in which the portion including the expandable-and-shrinkable member 11 is shrunk in the cross-machine direction CD. The shrinking step may also be included in the providing step.

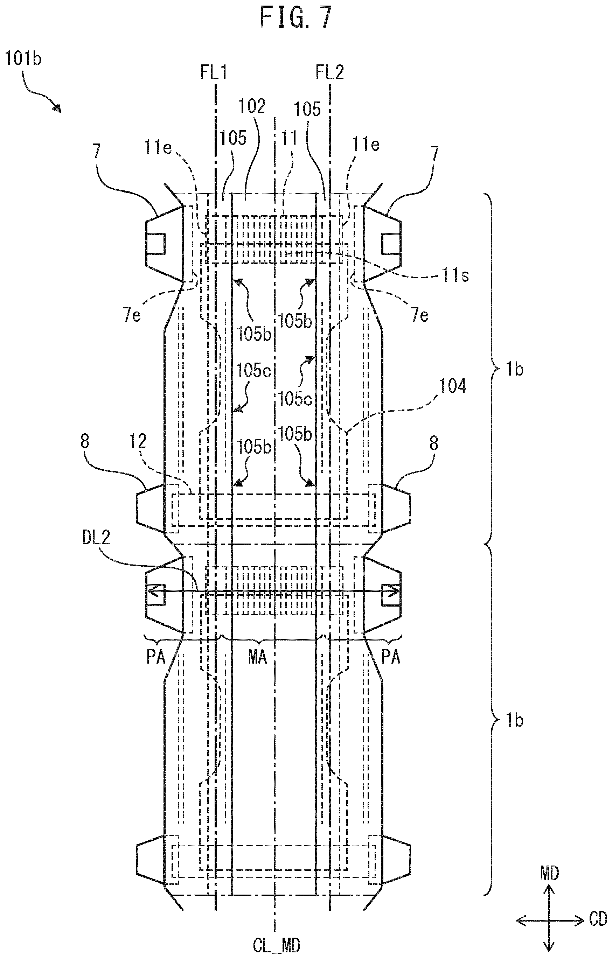

Fig. 7 schematically shows an example of the structure of a semi-processed continuous body 101b. The semi-processed continuous body 101b is composed of a plurality of semi-processed products 1b connected in the machine direction. One semi-processed product 1b has basically the same structure as the semi-processed product 1a shown in Fig. 6. Compared to the semi-processed product 1a, however, the portions including the expandable-and-shrinkable member 11 of the semi-processed product 1b are shrunk in the cross-machine direction CD (widthwise direction). That is, the reduced tension on the semi-processed continuous body 101a causes the expandable-and-shrinkable member 11 disposed in an expanded state in the cross-machine direction CD, to be shrunk in the cross-machine direction CD of the semi-processed product 1b. At the portions of the semi-processed continuous body 101b including the expandable-and-shrinkable member 11, the maximum length in the cross-machine direction CD (including the length of the protruding sections of the pair of protrusion sections 7, 7) DL2 is smaller than the length DL1 before shrink of the expandable-and-shrinkable member 11 (Fig. 6) (DL2 < DL1). At the same time, a plurality of wrinkles 11S are formed at the portions of the semi-processed continuous body 101b including the expandable-and-shrinkable member 11. The semi-processed continuous body 101b is transported by the folding unit 200F.

In the following pressing step, the semi-processed continuous body 101b with the expandable-and-shrinkable member 11 shrunk is transported in the machine direction MD, while a center region MA of the semi-processed product 1b in the cross-machine direction CD is pressed in the direction toward the mounting surface of the semi-processed product 1b (the transport surface of the endless belt 310b), with the pressing member 301 whose length in the cross-machine direction CD is smaller than the length of the expandable-and-shrinkable member 11 in the cross-machine direction CD. As a result, the region of the expandable-and-shrinkable member 11 with a plurality of wrinkles 11S becomes pressed.

In the folding step, the semi-processed continuous body 101b is transported in the machine direction MD while a pair of side section regions PA, PA on the outer sides of the center region MA of the semi-processed product 1b in the cross-machine direction CD are raised in the direction opposite from the direction toward the mounting surface of the semi-processed product 1b by the folding member 302 (guides 302a, 302a) on a pair of folding lines FL1, FL2 extending along the machine direction MD, and each is folded upward above the center region MA. A semi-processed continuous body 101c is thus formed. However, the pair of folding lines FL1, FL2 is set so as to extend along the machine direction MD, to the inner side from both edge lines 11e, 11e of the expandable-and-shrinkable member 11 in the cross-machine direction CD. They are preferably set so as to extend along the machine direction MD, to the inner side from the edge lines 7e, 7e on the inner side in the cross-machine direction CD of the pair of protrusion sections 7, 7. The pressing step and the folding step may be carried out in a temporally overlapping manner, or the folding step may be carried out after the pressing step.

The semi-processed product 1b has the pair of protrusion sections 7, 7 that are discontinuous in the machine direction MD, formed so as to protrude to the outer sides at both ends in the cross-machine direction CD (widthwise direction), and at the folding apparatus 300, the discontinuous pair of protrusion sections 7, 7 can potentially be difficult to fold in a stable manner. According to this embodiment, however, having the configuration described above using the expandable-and-shrinkable member 11 allows the pair of protrusion sections 7, 7 to be stably folded. The reason for this is as follows.

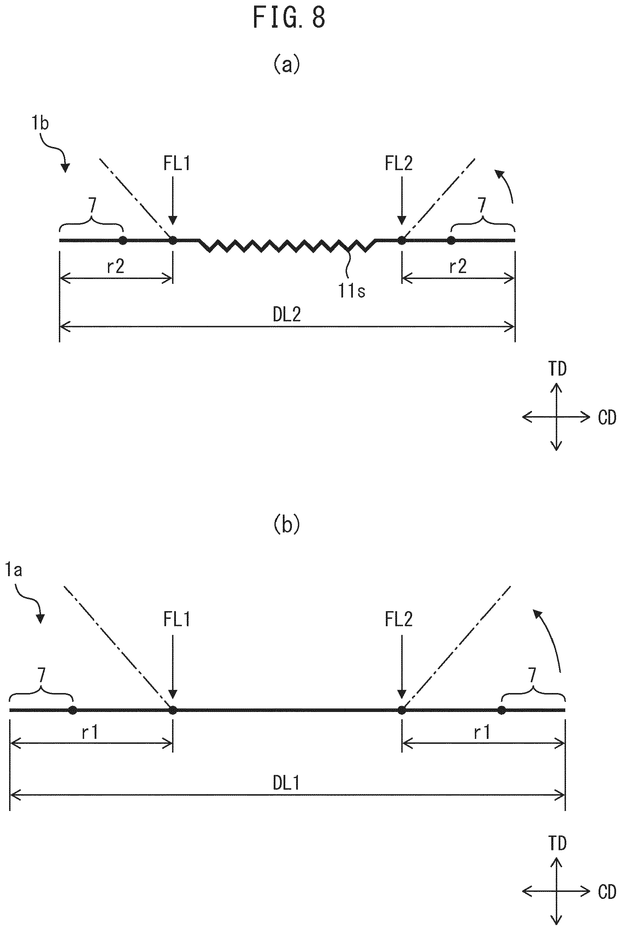

Fig. 8 is a schematic diagram illustrating a method of folding the semi-processed product 1b. Fig. 8(a) shows a case having the expandable-and-shrinkable member 11 where the expandable-and-shrinkable member 11 is shrunk, i.e., a case of the present embodiment. Fig. 8(b) shows a case not having the expandable-and-shrinkable member 11, i.e., a case that is not the present embodiment. When the expandable-and-shrinkable member 11 is not present, i.e., in the case that is not the present embodiment (Fig. 8(b)), the length in the cross-machine direction CD (widthwise direction) of the semi-processed product 1b is a relatively large value of DL1, while the lengths of the portions on the outer sides from the folding lines FL1, FL2 are also relatively large values of r1. Thus, moments of inertia around the folding lines FL1, FL2, of the portions (including the protrusion sections 7, 7) that are more toward the outer sides than the folding lines FL1, FL2, become relatively large values. On the other hand, when the expandable-and-shrinkable member 11 is present and shrunk, i.e., in the case of the present embodiment (Fig. 8(a)), the function of the expandable-and-shrinkable member 11 causes the length in the cross-machine direction CD (widthwise direction) of the portion including the expandable-and-shrinkable member 11 of the semi-processed product 1b to be a relatively small value of DL2 (<DL1), while the lengths of the portions on the outer sides from the folding lines FL1, FL2 are also relatively small values of r2 (<r1). Thus, the moments of inertia around the folding lines FL1, FL2, of the portions (including the protrusion sections) that are more toward the outer sides than the folding lines FL1, FL2, can become relatively small values. Thus, for the present embodiment, the force necessary to rotate the portions (including the protrusion sections) that are more toward the outer sides than the folding lines FL1, FL2, around the folding lines FL1, FL2 as the centers of rotation, i.e., the force necessary to raise them, can be reduced. As a result, the discontinuous protrusion sections 7, 7 can be easily raised.

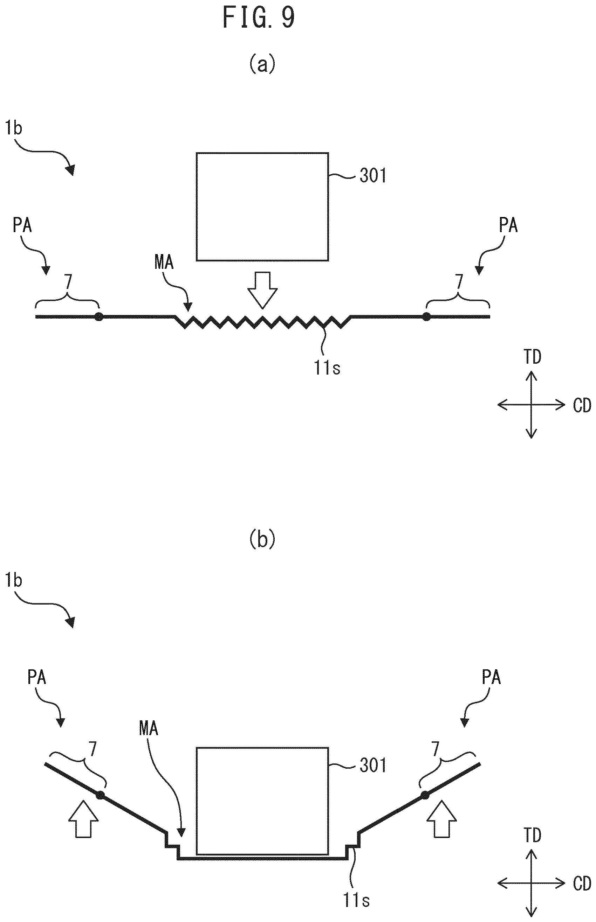

Fig. 9 is a schematic diagram illustrating a method of folding the semi-processed product 1b. Fig. 9(a) and Fig. 9(b) show the state of the portion including the expandable-and-shrinkable member 11 of the semi-processed product 1b before and after pressing by the pressing member 301, respectively. Since a plurality of wrinkles 11S are formed in the portion including the expandable-and-shrinkable member 11 due to the function of the expandable-and-shrinkable member 11, at least some of the plurality of wrinkles 11S are collapsed by the pressing member 301 when the center region MA of the semi-processed product 1b is pressed in the direction toward the mounting surface of the semi-processed product 1b by the pressing member 301 having a smaller length in the cross-machine direction CD (widthwise direction) than the expandable-and-shrinkable member. As a result, the tops of the wrinkles 11S that have convex shapes toward the side opposite the mounting surface side are pressed against the mounting surface side, and the wrinkles 11S are expanded, and thus the pressed regions spread out in the cross-machine direction CD on the mounting surface side. As the pressed regions spread out in the cross-machine direction CD on the mounting surface side, the non-pressed regions, i.e., the side section regions PA further on the outer sides in the cross-machine direction CD than the pressed regions, instead rise up in the direction opposite the direction toward the mounting surface. Consequently, the portion including the expandable-and-shrinkable member 11 of the semi-processed product 1b has, throughout, concave shapes formed on the side opposite the mounting surface. As a result, the protrusion sections 7, 7 disposed on both sides in the cross-machine direction CD of the side section regions PA also easily tend to rise to follow the concave shapes.

In this method for producing an absorbent article, by provision of the expandable-and-shrinkable member 11 in the semi-processed product (absorbent article), at least the two functions described above act synergistically to allow the protrusion sections 7, 7 that are discontinuous in the machine direction MD to be easily raised, thereby allowing the protrusion sections 7, 7 to be stably folded together with the side section regions PA, PA. In this case, the portions that have been folded need only be opened at one location on each side during use of the absorbent article 1, and therefore the burden of operation for the user is not increased. In other words, it is possible to stably fold the protrusion sections 7, 7 together with the side section regions PA, PA without increasing the burden of operation during use.

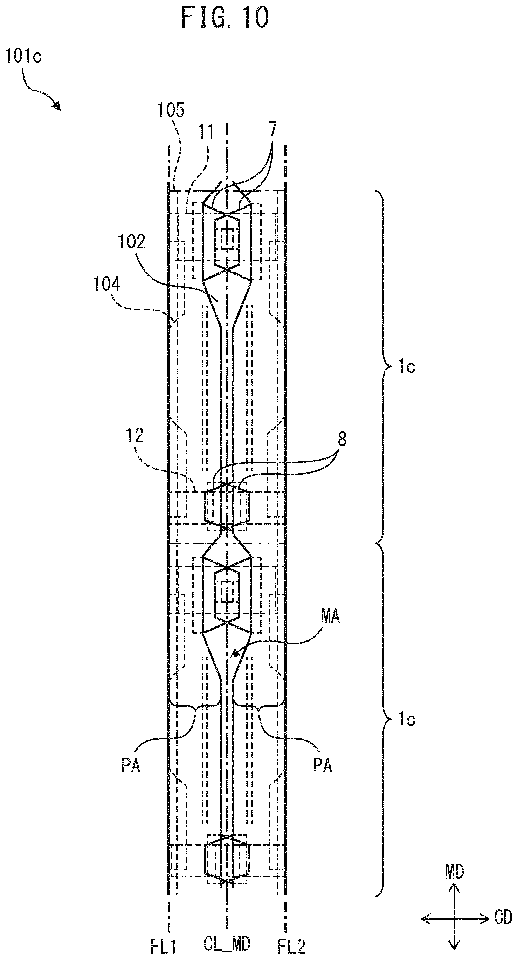

Fig. 10 schematically shows an example of the structure of a semi-processed continuous body 101c. The semi-processed continuous body 101c is composed of a plurality of semi-processed products 1c connected in the machine direction. One semi-processed product 1c has basically the same structure as the semi-processed product 1b shown in Fig. 7. However, compared to the semi-processed product 1b, the pair of side section regions PA, PA of the semi-processed product 1c are each folded upward over the center region MA.

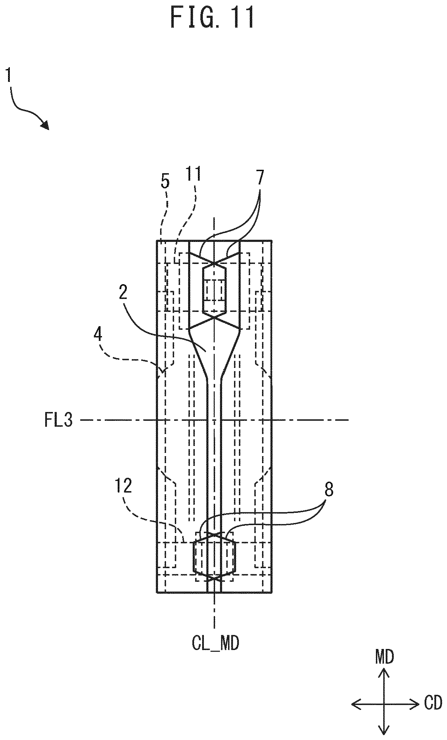

In the folding step, the semi-processed continuous body 101c is further cut into individual absorbent article product portions as the absorbent article 1. Fig. 11 schematically shows an example of the structure of an absorbent article 1 cut into a product portion. The cut absorbent article 1 has the same structure as the semi-processed product 1c of Fig. 8. Next, the absorbent article 1 is folded in the machine direction MD at a folding line FL3 that extends through the center section in the machine direction MD and extends along the cross-machine direction CD.

The absorbent article is produced in this manner.

According to the method for producing an absorbent article of the present embodiment described above, it is possible to stably fold the protrusion sections 7, 7 together with the side section regions PA, PA, without increasing the burden of operation during use.

As a preferred configuration in the method for producing an absorbent article according to this embodiment, the entire expandable-and-shrinkable member 11 overlaps with the pair of protrusion sections 7, 7 in the machine direction MD. It is thus possible to more powerfully exhibit the effect of mounting the expandable-and-shrinkable member 11 (the effect of lowering the moment of inertia of the portions including the protrusion sections around the folding lines FL1, FL2 and the effect of forming the expandable-and-shrinkable member 11 into a concave shape by pressing of the wrinkles 11S). The protrusion sections can thus be more stably folded together with the side section regions.

As another preferred configuration in the method for producing an absorbent article according to this embodiment, the folding lines FL1, FL2 overlap the regions where the rising sections 5c of the leak resistant walls 5 are overlapping and the rigidity is increased. This allows the rigidity to be increased at the creases by the folding lines FL1, FL2, to allow the creases to be stabilized. In addition, it can aid in rising of the side section regions PA, PA due to the shrink of the rising sections 5c in the machine direction MD. This allows the protrusion sections to be more stably folded together with the side section regions. As another preferred configuration, the rising sections 5c of the leak resistant walls 5 do not overlap the expandable-and-shrinkable member 11 in the machine direction MD. Thus, expansion-and-shrink of the expandable-and-shrinkable member 11 in the cross-machine direction CD is not affected by expansion of the rising sections 5c in the machine direction MD. This allows suitable shrink of the expandable-and-shrinkable member 11, and allows the effect of mounting the expandable-and-shrinkable member 11 to be more strongly exhibited. The protrusion sections can thus be more stably folded together with the side section regions. As yet another preferred configuration, the folding lines FL1, FL2 overlap the regions where the joint sections 5b of the leak resistant walls 5 overlap and the rigidity is increased. This allows the rigidity to be increased at the creases by the folding lines FL1, FL2, to allow the creases to be stabilized. The protrusion sections can thus be more stably folded together with the side section regions.

As another preferred configuration in the method for producing an absorbent article according to this embodiment, the folding lines FL1, FL2 overlap the regions where the to-be-connected member 12 overlaps and the rigidity is increased. This allows the rigidity to be increased at the creases by the folding lines FL1, FL2, to allow the creases to be stabilized. The protrusion sections can thus be more stably folded together with the side section regions. As yet another preferred configuration, the folding lines FL1, FL2 overlap the regions where the absorbent body 4 overlaps and the rigidity is increased. This allows the rigidity to be increased at the creases by the folding lines FL1, FL2, to allow the creases to be stabilized. The protrusion sections can thus be more stably folded together with the side section regions.

According to yet another preferred configuration of the method for producing an absorbent article, the folding lines FL1, FL2 may be present in the region of the wrinkles 11S of the expandable-and-shrinkable member 11. In the folding step, the side section regions PA, PA can be easily folded with the wrinkles 11S as the origin, whereby the protrusion sections can be more stably folded together with the side section regions. According to yet another preferred configuration, the expandable-and-shrinkable member 11 and the protrusion sections 7, 7 may be overlapping. This allows the rigidity to be increased in the portions from the expandable-and-shrinkable member 11 to the protrusion sections 7, 7. The protrusion sections can thus be more stably folded together with the side section regions.

### Reference Signs List

1 Absorbent article
1a, 1b, 1c Semi-processed products
7, 8 Protrusion section
11 Expandable-and-shrinkable member
11S Wrinkles
101a, 101b, 101c Semi-processed continuous body

## Claims

1. A method for producing an absorbent article (1), comprising:
a providing step of providing a semi-processed continuous body comprising semi-processed products (1b) for the absorbent article connected in a machine direction, the semi-processed product comprising a pair of protrusion sections formed protruding outward at both ends in the widthwise direction of the semi-processed product, and an expandable-and-shrinkable member (11) that is expandable and shrinkable in an expansion-and-shrink direction and is disposed in an expanded state between the pair of protrusion sections so that the expansion-and-shrink direction is along the widthwise direction;
**characterised by** a shrinking step of shrinking the expandable-and-shrinkable member to reduce a length in the widthwise direction at a portion including the expandable-and-shrinkable member in the semi-processed product to a length shorter than that before the shrinking, and form a plurality of wrinkles (115) in the expandable-and-shrinkable member;
a pressing step of pressing a center region in the widthwise direction of the semi-processed product, while transporting the semi-processed continuous body with the expandable-and-shrinkable member shrunk in the machine direction; and
a folding step of raising, in a direction opposite from a direction of the pressing, a pair of side section regions (PA, PA) on both outer sides in the widthwise direction of the center region of the semi-processed product at a pair of folding lines (FL1, FL2) extending along the machine direction set more toward an inner side than both end sections in the widthwise direction of the expandable-and-shrinkable member, and folding each of the pair of side section regions upward above the center region, while transporting the semi-processed continuous body in the machine direction.

2. The method for producing an absorbent article according to claim 1, wherein the folding lines are set extending through regions with the plurality of wrinkles.

3. The method for producing an absorbent article according to claim 1 or 2, wherein the entire expandable-and-shrinkable member overlaps the pair of protrusion sections in the machine direction.

4. The method for producing an absorbent article according to any one of claims 1 to 3, wherein the semi-processed product comprises a pair of leak resistant walls extending along the machine direction and disposed separately in the widthwise direction,
the leak resistant walls includes rising sections which are folded so as to be capable of rising in a thickness direction of the semi-processed product, and in which edges on inner sides in the widthwise direction are free ends so as to be expandable and shrinkable and edges on outer sides in the widthwise direction are fixed ends, and
the folding lines are set so as to extend through the rising sections.

5. The method for producing an absorbent article according to claim 4, wherein the rising sections do not overlap the expandable-and-shrinkable member in the machine direction.

6. The method for producing an absorbent article according to claim 4 or 5, wherein the leak resistant walls comprise joint sections extending in the machine direction, connecting the rising sections to the semi-processed product at both ends in the machine direction of the rising sections, and
the folding lines are set so as to extend through the joint sections.

7. The method for producing an absorbent article according to any one of claims 1 to 6, wherein the semi-processed product further comprises:
another pair of protrusion sections disposed at both ends in the widthwise direction protruding toward the outer sides in the widthwise direction, and
a to-be-connected member disposed between the other pair of protrusion sections, that is a member to which the pair of protrusion sections is joined during use of the absorbent article, and
the folding lines are set so as to extend through the to-be-connected member.

8. The method for producing an absorbent article according to any one of claims 1 to 7, wherein inner side edges of the pair of protrusion sections in the widthwise direction and outer side edges of the expandable-and-shrinkable member in the widthwise direction are mutually overlapping.

9. The method for producing an absorbent article according to any one of claims 1 to 8, wherein the semi-processed product further comprises:
an absorbent body disposed extending through a center in the widthwise direction and along the machine direction, and
the folding lines are set so as to extend through edges in the widthwise direction of the absorbent body.

## Patentansprüche

1. Verfahren für die Herstellung eines absorbierenden Artikels (1), das Folgendes umfasst:
einen Bereitstellungsschritt zur Bereitstellung eines halbverarbeiteten kontinuierlichen Körpers, der halbverarbeitete Produkte (1b) für den absorbierenden Artikel umfasst, die in einer Maschinenlaufrichtung verbunden sind, wobei das halbverarbeitete Produkt Folgendes umfasst: ein Paar an überstehenden Abschnitten, die nach außen überstehend an beiden Enden in der Breitenrichtung des halbverarbeiteten Produkts ausgebildet sind, und ein erweiterbares und schrumpfbares Element (11), das in einer Erweiterung-und-Schrumpfung-Richtung erweiterbar und schrumpfbar ist und in einem erweiterten Zustands zwischen dem Paar an überstehenden Abschnitten derart angeordnet ist, dass die Erweiterung-und-Schrumpfung-Richtung entlang der Breitenrichtung verläuft;
**gekennzeichnet durch**: einen Schrumpfungsschritt zur Schrumpfung des erweiterbaren und schrumpfbaren Elements, um eine Länge in der Breitenrichtung an einem Teil, dass das erweiterbare und schrumpfbare Element in dem halbverarbeiteten Produkt einschließt, auf eine Länge zu verringern, die kürzer ist als die vor der Schrumpfung, und eine Vielzahl von Falten (115) in dem erweiterbaren und schrumpfbaren Element zu bilden;
einen Pressschritt zum Pressen eines mittleren Bereichs in der Breitenrichtung des halbverarbeiteten Produkts während des Transportierens des halbverarbeiteten kontinuierlichen Körpers mit dem geschrumpften erweiterbaren und schrumpfbaren Element in der Maschinenlaufrichtung; und
einen Faltschritt zum Anheben in einer Richtung, entgegengesetzt von einer Richtung des Pressens, eines Paars an Seitenabschnittsbereichen (PA, PA) an beiden äußeren Seiten in der Breitenrichtung des mittleren Bereichs des halbverarbeiteten Produkts an einem Paar an Faltlinien (FL1, FL2), die sich entlang der Maschinenlaufrichtung erstrecken, mehr zu einer inneren Seite als beide Endabschnitte in der Breitenrichtung des erweiterbaren und schrumpfbaren Elements festgelegt sind, und zum Falten von jedem des Paars an Seitenabschnittsbereichen nach oben über den mittleren Bereich während des Transportierens des halbverarbeiteten kontinuierlichen Körpers in der Maschinenlaufrichtung.

2. Verfahren für die Herstellung eines absorbierenden Artikels nach Anspruch 1, wobei die Faltlinien festgelegt sind, dass sie sich durch Bereiche mit einer Vielzahl von Falten erstrecken.

3. Verfahren für die Herstellung eines absorbierenden Artikels nach Anspruch 1 oder 2, wobei das gesamte erweiterbare und schrumpfbare Element das Paar an überstehenden Abschnitten in der Maschinenlaufrichtung überlappt.

4. Verfahren für die Herstellung eines absorbierenden Artikels nach einem der Ansprüche 1 bis 3, wobei das halbverarbeitete Produkt ein Paar an auslaufbeständigen Wänden umfasst, die sich entlang der Maschinenlaufrichtung erstrecken und separat in der Breitenrichtung angeordnet sind,
die auslaufbeständigen Wände hochragende Abschnitte einschließen, die derart gefaltet werden, dass sie in einer Dickenrichtung des halbverarbeiteten Produkts hochragen können, und in welchen Ränder auf inneren Seiten in der Breitenrichtung freie Enden sind, sodass sie erweiterbar und schrumpfbar sind, und Ränder an äußeren Seiten in der Breitenrichtung fixierte Enden sind, und
die Faltlinien derart festgelegt sind, dass die sich durch die hochragenden Abschnitte erstrecken.

5. Verfahren für die Herstellung eines absorbierenden Artikels nach Anspruch 4, wobei die hochragenden Abschnitte nicht das erweiterbare und schrumpfbare Element in der Maschinenlaufrichtung überlappen.

6. Verfahren für die Herstellung eines absorbierenden Artikels nach Anspruch 4 oder 5, wobei die auslaufbeständigen Wände Verbindungsabschnitte umfassen, die sich in der Maschinenlaufrichtung erstrecken, die die hochragenden Abschnitte mit dem halbverarbeiteten Produkt an beiden Enden in der Maschinenlaufrichtung der hochragenden Abschnitte verbinden, und
die Faltlinien derart festgelegt sind, dass sie sich durch die Verbindungsabschnitte erstrecken.

7. Verfahren für die Herstellung eines absorbierenden Artikels nach einem der Ansprüche 1 bis 6, wobei das halbverarbeitete Produkt weiter Folgendes umfasst:
ein anderes Paar an überstehenden Abschnitten, die an beiden Enden in der Breitenrichtung angeordnet sind, zu den äußeren Seiten in der Breitenrichtung überstehen, und
ein zu verbindendes Element, das zwischen dem anderen Paar an überstehenden Abschnitten angeordnet ist, das ein Element ist, mit dem das Paar an überstehenden Abschnitten während der Verwendung des absorbierenden Artikels verbunden wird, und
die Faltlinien derart festgelegt sind, dass sie sich durch das zu verbindende Element erstrecken.

8. Verfahren für die Herstellung eines absorbierenden Artikels nach einem der Ansprüche 1 bis 7, wobei Ränder der inneren Seite des Paars an überstehenden Abschnitten in der Breitenrichtung und Ränder der äußeren Seite des erweiterbaren und schrumpfbaren Elements in der Breitenrichtung gegenseitig überlappen.

9. Verfahren für die Herstellung eines absorbierenden Artikels nach einem der Ansprüche 1 bis 8, wobei das halbverarbeitete Produkte weiter Folgendes umfasst:
einen Saugkörper, der, sich durch eine Mitte in der Breitenrichtung und entlang der Maschinenlaufrichtung erstreckend, angeordnet ist, und
die Faltlinien derart festgelegt sind, dass sie sich durch Ränder in der Breitenrichtung des Saugkörpers erstrecken.

## Revendications

1. Procédé de production d'un article absorbant (1), comportant :
une étape de mise en oeuvre consistant à mettre en oeuvre un corps continu semi-fini comportant des produits semi-finis (1b) pour l'article absorbant qui sont reliés dans une direction allant dans le sens machine, le produit semi-fini comportant une paire de sections formant parties saillantes formées pour faire saillie vers l'extérieur au niveau des deux extrémités dans la direction allant dans le sens de la largeur du produit semi-fini, et un élément en mesure de s'étendre et de rétrécir (11) qui est en mesure de s'étendre et de rétrécir dans une direction allant dans le sens de l'extension et du rétrécissement et qui est disposé dans un état étendu entre la paire de sections formant parties saillantes de telle sorte que la direction allant dans le sens de l'extension et du rétrécissement se trouve le long de la direction allant dans le sens de la largeur ;
**caractérisé par** une étape de rétrécissement consistant à faire rétrécir l'élément en mesure de s'étendre et de rétrécir pour réduire une longueur dans la direction allant dans le sens de la largeur au niveau d'une partie comprenant l'élément en mesure de s'étendre et de rétrécir dans le produit semi-fini en une longueur plus courte que celle avant le rétrécissement, et pour former une pluralité de fronces (115) dans l'élément en mesure de s'étendre et de rétrécir ;
une étape de compression consistant à comprimer une région centrale dans la direction allant dans le sens de la largeur du produit semi-fini, tout en transportant le corps continu semi-fini avec l'élément en mesure de s'étendre et de rétrécir rétréci dans la direction allant dans le sens machine ; et
une étape de pliage consistant à lever, dans une direction opposée à une direction de la compression, une paire de régions formant sections latérales (PA, PA) sur les deux côtés extérieurs dans la direction allant dans le sens de la largeur de la région centrale du produit semi-fini au niveau d'une paire de lignes de pliage (FL1, FL2) s'étendant le long de la direction allant dans le sens machine se trouvant plus vers un côté intérieur par rapport aux deux sections d'extrémité dans la direction allant dans le sens de la largeur de l'élément en mesure de s'étendre et de rétrécir, et consistant à plier chacune de la paire de régions formant sections latérales vers le haut au-dessus de la région centrale, tout en transportant le corps continu semi-fini dans la direction allant dans le sens machine.

2. Procédé de production d'un article absorbant selon la revendication 1, dans lequel les lignes de pliage sont réglées pour s'étendre au travers de régions avec la pluralité de fronces.

3. Procédé de production d'un article absorbant selon la revendication 1 ou la revendication 2, dans lequel l'élément en mesure de s'étendre et de rétrécir tout entier chevauche la paire de sections formant parties saillantes dans la direction allant dans le sens machine.

4. Procédé de production d'un article absorbant selon l'une quelconque des revendications 1 à 3, dans lequel le produit semi-fini comporte une paire de parois résistantes aux fuites s'étendant le long de la direction allant dans le sens machine et disposées séparément dans la direction allant dans le sens de la largeur,
les parois résistantes aux fuites comprennent des sections de levage qui sont pliées de manière à être en mesure de se lever dans une direction allant dans le sens de l'épaisseur du produit semi-fini, et dans lesquelles des bords sur des côtés intérieurs dans la direction allant dans le sens de la largeur sont des extrémités libres de manière à être en mesure de s'étendre et de rétrécir et des bords sur des côtés extérieurs dans la direction allant dans le sens de la largeur sont des extrémités fixes, et
les lignes de pliage sont réglées de manière à s'étendre au travers des sections de levage.

5. Procédé de production d'un article absorbant selon la revendication 4, dans lequel les sections de levage ne chevauchent pas l'élément en mesure de s'étendre et de rétrécir dans la direction allant dans le sens machine.

6. Procédé de production d'un article absorbant selon la revendication 4 ou la revendication 5, dans lequel les parois résistantes aux fuites comportent des sections formant joints s'étendant dans la direction allant dans le sens machine, servant à relier les sections de levage au produit semi-fini aux deux extrémités dans la direction allant dans le sens machine des sections de levage, et
les lignes de pliage sont réglées de manière à s'étendre au travers des sections formant joints.

7. Procédé de production d'un article absorbant selon l'une quelconque des revendications 1 à 6, dans lequel le produit semi-fini comporte par ailleurs :
une autre paire de sections formant parties saillantes disposées aux deux extrémités dans la direction allant dans le sens de la largeur faisant saillie vers les côtés extérieurs dans la direction allant dans le sens de la largeur, et
un élément destiné à être relié disposé entre l'autre paire de sections formant parties saillantes, qui est un élément auquel la paire de sections formant parties saillantes est jointe lors de l'utilisation de l'article absorbant, et
les lignes de pliage sont réglées de manière à s'étendre au travers de l'élément destiné à être relié.

8. Procédé de production d'un article absorbant selon l'une quelconque des revendications 1 à 7, dans lequel des bords des côtés intérieurs de la paire de sections formant parties saillantes dans la direction allant dans le sens de la largeur et des bords des côtés extérieurs de l'élément en mesure de s'étendre et de rétrécir dans la direction allant dans le sens de la largeur se chevauchent mutuellement.

9. Procédé de production d'un article absorbant selon l'une quelconque des revendications 1 à 8, dans lequel le produit semi-fini comporte par ailleurs :
un corps absorbant disposé pour s'étendre au travers d'un centre dans la direction allant dans le sens de la largeur et le long de la direction allant dans le sens machine, et
les lignes de pliage sont réglées de manière à s'étendre au travers des bords dans la direction allant dans le sens de la largeur du corps absorbant.
